(19) **Europäisches Patentamt European Patent Office Office européen des brevets**

(11) **EP 4 091 707 B1**

(12) **EUROPEAN PATENT SPECIFICATION**

(45) Date of publication and mention
of the grant of the patent:
**03.07.2024 Bulletin 2024/27**

(51) International Patent Classification (IPC):
**B01J 3/03** *(2006.01)* **B01J 19/00** *(2006.01)*
**C07D 251/60** *(2006.01)* **C07D 251/62** *(2006.01)*

(21) Application number: **22173981.6**

(22) Date of filing: **18.05.2022**

(52) Cooperative Patent Classification (CPC):
**B01J 3/03; B01J 3/046; B01J 19/006;**
**B01J 19/0073; C07D 251/60; C07D 251/62;**
B01J 2219/00162; B01J 2219/00768

(54) **APPARATUS FOR THE PRODUCTION OF MELAMINE**

APPARAT ZUR HERSTELLUNG VON MELAMIN

APPAREIL POUR LA PRODUCTION DE MÉLAMINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.05.2021 IT 202100013079**

(43) Date of publication of application:
**23.11.2022 Bulletin 2022/47**

(73) Proprietor: **Proman AG**
**8832 Wollerau Schwyz (CH)**

(72) Inventors:
• **DE AMICIS, Alberto**
**I-20097 San Donato Milanese (MI) (IT)**
• **DI RUOCCO, Giuseppe**
**I-23900 Lecco (IT)**
• **SANTUCCI, Roberto**
**I - 21050 Gorla Maggiore (VA) (IT)**

(74) Representative: **De Gregori, Antonella et al BIRD & BIRD SOCIETA TRA AVVOCATI S.R.L. Via Porlezza, 12 20123 Milano (IT)**

(56) References cited:
CN-A- 109 761 922    DE-A1- 10 319 902
US-A1- 2011 054 169    US-B2- 6 870 050

**Description**

The present invention relates to an apparatus for the production of melamine and a process for controlling and compensating the pressure differences in an apparatus for the production of melamine comprising at least one reactor and at least one post-reactor in the reaction section.

For a better understanding of the object of the present invention, reference is made to the state of the art as described hereunder.

It is known that the transformation of molten urea into melamine is described by the following overall reaction (1):

$$6 \ NH_2CONH_2 \rightarrow (CN)_3(NH_2)_3 + 6 \ NH_3 + 3 \ CO_2 \qquad (1)$$

urea melamine ammonia carbon dioxide wherein, for every kilogram of melamine, 1.86 kilograms of $NH_3$ and $CO_2$, globally referred to as off-gases, are formed.

As has been described numerous times, the off-gases coming from the conversion reaction of urea to melamine are recycled to a urea plant for their reconversion and therefore represent a supply stream to the urea plant which contains $NH_3$ and $CO_2$.

The reaction for the production of urea from $NH_3$ and $CO_2$ takes place through the following reaction:

$$2 \ NH_3 + 1 \ CO_2 \rightarrow 1 \ CO(NH_2)_2 + 1 \ H_2O \qquad (2)$$

ammonia carbon dioxide urea water

The molar ratio between $NH_3$ and $CO_2$ is equal to 2 and this ratio is that which is optimal for all the supply streams to the urea plant, including the off-gas stream from the melamine plant.

The ratio between $NH_3$ and $CO_2$ in the off-gas stream from the melamine plant depends on how much ammonia is fed to the melamine reaction section.

As can be seen from reaction (1) above, also in this case the $NH_3$ and $CO_2$ ratio is equal to 2 and this ratio is modified by the quantity of $NH_3$ that is fed into the bottom of the reactor and the post-reactor.

As described hereunder, it is important to feed $NH_3$ to the reactor and post-reactor in order to favour the conversion of the by-products to melamine in the reactor and strip the $CO_2$ from the raw melamine in the post-reactor. It is consequently always necessary, for the requirements of the melamine plant, to maintain a stream of $NH_3$ in the feeding to the reaction section.

It should also be remembered that in urea technologies, the supply of $NH_3$ and $CO_2$ to the urea reactor does not have a stoichiometric ratio of 2, but rather a ratio that varies according to the technologies themselves. All the technologies used industrially for the production of urea are with total recycling and, regardless of the $NH_3/CO_2$ ratio in the reactor, the $NH_3/CO_2$ ratio at the inlet of the plant is equal to 2. In order to satisfy the technological necessity of the urea reactor of operating with an $NH_3/CO_2$ molar ratio higher than the stoichiometric ratio, a series of recyclings between the different sections are applied in the different technologies in order to recycle the reagents in the reactor until they are converted into urea. In order to be optimal for the requirements of the urea plant, the off-gas stream from the melamine plant must have an $NH_3/CO_2$ ratio as close as possible to the stoichiometric ratio in order to limit the number of recyclings inside the urea plant.

Almost all of the latest-generation melamine plants, operating at high pressure with a pressure higher than 70 barg and a temperature above 360°C, adopt a reaction section comprising at least one reactor and at least one post-reactor (also called finishing converter or finisher or $NH_3$ stripper), from each of which at least two streams are extracted, a stream in the gaseous phase composed of $NH_3$, $CO_2$ and melamine in vapour phase and a stream of molten melamine, comprising by-products and reaction intermediates, and in which $NH_3$ and $CO_2$ remain in solution in equilibrium with their partial pressures.

In some plants, a solution is opted for which provides a single outlet from the reactor, which is equipped downstream with a separator, from which the above-mentioned gas and liquid phases exit separately.

The stream of molten raw melamine, exiting from the upper portion of the reactor, is fed into the upper portion of the post-reactor normally through communicating vessels, whereas the stream of purified melamine, which exits from the lower portion of the post-reactor by means of a level controller, is sent to the sections of the melamine plant downstream where it is treated according to different separation/purification processes.

The gaseous streams leaving the upper portion of the reactor and post-reactor, before being used in other processes, for example before being used for the production of urea, must be treated in order to separate and recover the melamine present in the same, at the same time recovering at least part of the heat contained therein.

There are mainly two methods used for this washing treatment (also called scrubbing): scrubbing the gaseous streams with aqueous solutions or scrubbing the gaseous streams with molten urea. After scrubbing, once the traces of melamine have been removed, the gaseous streams are called off-gases.

This scrubbing step does not create problems in the case of scrubbing with aqueous solutions as

- the heat contained in the gaseous streams is used for maintaining the operating temperature of the system and
- the aqueous scrubbing solution is always available as long as the sections downstream of the reaction section are running and
- the scrubbing solution itself can be recycled for a long time as it is not subject to degradation or saturation of melamine as this is continuously extracted in the sections downstream (i.e. in the crystallization sections).

In the case of scrubbing with molten urea, on the contrary, various problems must be considered: as molten urea is in fact subject to degradation reactions with the formation of by-products which, above certain concentrations, precipitate with the formation of a solid product and with the consequent clogging of the system, and as the degradation rate of urea is directly proportional to the temperature, the temperature must be controlled by recycling through a heat exchanger with the production of steam to prevent the urea, on excessively increasing the temperature, from rapidly degrading with the formation of by-products.

A further drawback of scrubbing with urea is that the quantity of melamine that may be present in dispersion in the molten scrubbing urea is limited by the necessity of avoiding its possible precipitation and the formation of encrustations.

It is consequently evident that the scrubbing urea cannot be kept in recycling without adequate renewal to avoid its saturation of both melamine and by-products.

It is evident that the urea used for scrubbing the gaseous streams leaving the reaction section, containing melamine, can only be used for the production of melamine, therefore it can only be recycled as a feed to the reactor for the synthesis of melamine: the quantity of urea that can be used for continuously renewing the urea circulating in the scrubbing section (i.e. the scrubbing urea) therefore corresponds to the quantity of urea used for scrubbing which is fed to the reactor. In the event of a stoppage in the supply of urea to the reactor, there is therefore also no possibility of renewing the scrubbing urea used for scrubbing the gas phases.

According to the state of the art, the two gaseous streams leaving the reactor and the post-reactor respectively are generally combined and scrubbed in a single scrubbing column in order to recover the melamine contained therein and then be sent to plants downstream for their recovery. The off-gases are normally sent to a urea plant as they mainly consist of $NH_3$ and $CO_2$.

The scrubbing operation is of primary importance not only for the recovery of the melamine contained in the gaseous streams, but above all for preventing said melamine in vapour phase, when not adequately absorbed and dispersed, or solubilized in the scrubbing medium, from exiting from the reaction section which is kept at temperatures higher than 360°C (preferably higher than 370°C), then condensing in cold areas of the system, causing progressive clogging.

It therefore appears essential that the gaseous streams exit from the reactor and post-reactor in the presence of the scrubbing fluid suitable for separating and absorbing the melamine contained therein. This aspect represents a considerable limitation in the case of scrubbing effected with urea as, even during the transitional phases in which no urea is fed to the reaction section, but the reactor and post-reactor are kept full for a rapid restart, a feed of $NH_3$ to the reaction section must be maintained. There is in any case a gaseous stream containing melamine that must be scrubbed and this operation cannot take place in the scrubbing column with preselected urea, as the scrubbing fluid is lacking, but resort must be made to alternative methods or the reaction section must be rapidly emptied. These transitory phases in which urea is not fed to the reaction section, with the necessity however of maintaining a supply of $NH_3$ to the section itself, are for example cases of $NH_3$ used in various flushings to avoid possible clogging of the lines and allowing a rapid restart.

The same problem arises with greater significance during the emptying of one or more of the apparatuses of the reaction section of the plant by draining or sublimating the melamine contained therein and flushing for the complete emptying of the section (s) before proceeding with the opening of the apparatuses and their cleaning: in this case, as the emptying rate by sublimation is proportional to the quantity of ammonia fed, the quantity of ammonia used is certainly a significant quantity which can be higher than 1,000 kg/h. In addition, the emptying operation by sublimation of the melamine lasts for several days.

For the correct functioning of a plant such as that previously considered, wherein the molten melamine is transferred from the reactor to the post-reactor through communicating vessels, it is of primary importance that the pressure in the two apparatuses, reactor and post-reactor, be the same in order to avoid, in the case of a higher pressure in the reactor, there being a passage of the gaseous stream/phase from the reactor to the post-reactor, causing an increase in the concentration of $CO_2$ in the gaseous stream/ phase of the post-reactor or, in the opposite case, that the melamine is no longer able to enter the post-reactor causing a level rise inside the reactor until the molten melamine exits with the gaseous stream/phase.

Consequently, in order to avoid the generation of a pressure difference between reactor and post-reactor, the two respective gaseous streams have so far always been treated jointly both in the case of scrubbing with aqueous solution and in the case of scrubbing with molten urea. It is in fact easy to regulate, by means of a single pressure regulator, the pressure of these two combined streams, simultaneously regulating in parallel the pressure of the reactor and the pressure of the post-reactor: as the two gaseous streams/phases of the reactor and post-reactor are in communication with each other through the outlet pipe of the gaseous stream/phase, the internal pressure that weighs on the surfaces of the liquid phases in the reactor and in the post-reactor will be equal.

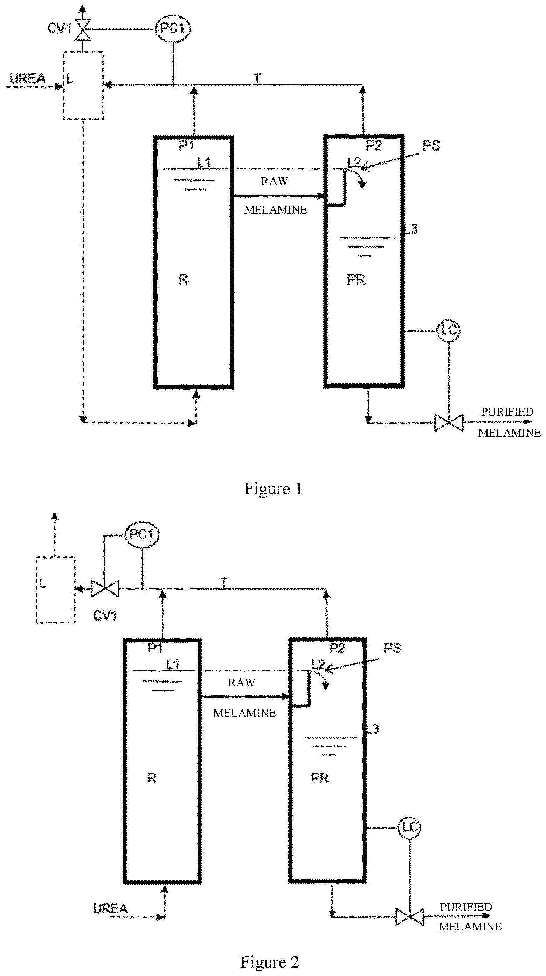

To clarify the above, reference is made to the attached figures 1 and 2 which represent the reaction and scrubbing section of a plant according to the state of the art wherein, in figure 1, a plant with scrubbing of the gaseous streams/phases with urea is represented. and in figure 2, a plant with scrubbing of the gaseous streams/phases with aqueous solution.

L1 is the level of the liquid phase in the reactor (R), L2 is the spillway level of the hydraulic seal at the spillway point (PS) in the post-reactor (PR), T is the outlet pipe of the gaseous streams/phases, P1 is the internal pressure of the reactor and P2 is the internal pressure of the post-reactor: in the apparatus of figure 1 and figure 2, P1 is equal to P2 and it follows that L1 is equal to L2.

As shown in figures 1 and 2 and as already mentioned, the liquid phases of the reactor and post-reactor are in communication by means of a pipe which continuously transfers the melamine produced in the reactor to the post-reactor through communicating vessels, whose level stability is guaranteed by the equal pressure of the gaseous streams/phases.

More specifically, figure 1 shows the situation of the state of the art wherein the gas phases leaving the reaction section (from R and PR) are scrubbed with molten urea (the scrubbing section (L) is indicated only as a whole), the raw melamine produced in the reactor (R) exits from the upper part of the reactor (R) through an overflow nozzle and is conveyed, by means of a pipe that will always be filled with only the liquid phase, in the upper part of the post-reactor (PR) at the base of a hydraulic seal from whose top (PS) the melamine exits and thus enters the post-reactor (PR). In the post-reactor, the level of the liquid phase is always kept, by means of a level controller, lower than the top of the hydraulic seal; in this way, interference between the level of the liquid phase in the post-reactor and the hydraulic seal is avoided, ensuring a constant height of the liquid column at the point of entry of the melamine into the post-reactor.

Said hydraulic seal, in fact, maintains a constant height of the liquid phase in its interior and in a higher position with respect to the point of entry of the melamine into the post-reactor, i.e. above the outlet nozzle of the reactor; said nozzle will be submerged and passed through by the liquid phase of the reactor, at the same time preventing the gas phase of the reactor from entering the post-reactor.

The pressures of the reactor and post-reactor, both regulated by the same controller, PC1, and by a single valve, CV1, which can be positioned both upstream and downstream of the scrubbing section with molten urea, will in fact be equal and consequently, based on the principle of the communicating vessels, the liquid phases connected to each other, L1 and L2, will be at the same level or height (the minimum differences due to the pressure drop of the connection pipe between the reactor and post-reactor are insignificant based on the ratio between the flow-rate and pipe diameter); the melamine produced will tend to accumulate above the liquid surface of L1 causing an increase in the internal level of the liquid phase of the reactor which will immediately result in a thrust of the liquid from the reactor to the post-reactor with the exiting of the melamine from the top of the hydraulic seal (PS) inside the post-reactor.

Figure 2 shows the state of the art with a reaction section equal to that described for figure 1, but with the scrubbing of the gas phases carried out with aqueous solution. In this case the urea is fed directly to the reactor and not before to the scrubbing section as in figure 1.

It should be noted that the two gas phases leaving the reactor and the post-reactor, although having the same components, completely differ in the concentrations of $NH_3$ and $CO_2$.

The compositions leaving the reactor and the post-reactor are different as the functions of the reactor and the post-reactor are different. The almost total conversion of urea into melamine takes place in the reactor, this reaction generates six moles of $NH_3$ and three moles of $CO_2$ for each mole of melamine. In the post-reactor, on the other hand, the raw melamine leaving the reactor is kept under the same conditions as the reactor in order to convert any remaining urea into melamine and complete the transformation of the reaction intermediates. A second important function of the post-reactor is to strip the $CO_2$ dissolved therein from the raw melamine. For this purpose, a superheated stream of $NH_3$ is fed into the bottom of the post-reactor which acts as a stripping fluid for the removal of the $CO_2$.

From what has been described above, it is evident that the composition of the gas phase coming from the reactor derives mainly from $NH_3$ and $CO_2$ generated in the conversion reaction of urea into melamine, whereas the gas phase coming from the post-reactor derives mainly from the $NH_3$ fed into the bottom of the post-reactor and from the stripped $CO_2$.

In the light of these differences, the possibility of treating the individual gas phases separately to obtain a greater benefit in economic terms of the plant management, has however been taken into consideration by the state of the art and described in EP3597641 B1. This document, however, does not describe how to solve the above-mentioned problem of keeping the pressures of the gas phase in the reactor and in the post-reactor the same, these pressures being controlled with two different pressure controllers and which can therefore be subject to variations due to both different response rates of the two control systems (controller and regulating valve) and also reading misalignments of the control systems themselves; it should be remembered that pressure differences on the surface of the liquid phase affect the level balance maintained through communicating vessels.

It should be considered, however, that with modern instrumental control systems, high reliability of both measurement and control has been achieved and detectable pressure variations have now been reduced to fractions of units. Some further examples of devices for production of melamine, comprising a melamine reactor and a post reactor, are disclosed in DE10319902A1, US2011/054169A1, CN109761922A and US6870050B2.

The objective of the present invention is to overcome the drawbacks of the state of the art previously described, defining an apparatus and a method for balancing and compensating the pressure differences that are generated between reactor and post-reactor in the case of the separate scrubbing of the two gas-phase streams, particularly advantageous in particular in the case of the scrubbing of the two gas-phase streams with urea. These and other objectives are achieved by means of the apparatus and the process according to the present invention.

The present invention therefore relates to an apparatus for the production of melamine which comprises a reaction section and a scrubbing section, wherein:

- said reaction section comprises at least one reactor (R) and at least one post-reactor (PR);
- said scrubbing section (L) is suitable for scrubbing the gas phase coming from the reactor with an aqueous solution or is suitable for scrubbing the gas phase coming from the reactor with urea;
- said reactor is connected to the scrubbing section through a means (T1) suitable for transferring the gaseous phase from the reactor to the scrubbing section;
- said post-reactor is connected through a means (T2) suitable for transferring the gaseous phase from the post-reactor to sections downstream;
- said means T1 and T2 are distinct, or communicating with at least one shut-off valve (B) positioned between the intersection point between said means (T1) and the outlet duct (U1) of the gas phase from the reactor and the intersection point between said means (T2) and the outlet duct (U2) of the gas phase from the post-reactor;
- said apparatus comprises two separate pressure-controller systems (PC1/CV1, PC2/CV2) suitable for controlling the pressure inside the reactor and inside the post-reactor respectively, each comprising a shut-off element (CV1, CV2) and a pressure-control means (PC1, PC2);
- said reactor and post-reactor are connected through a means (M) suitable for transferring the melamine from said reactor to said post-reactor, said means (M) connecting the upper portion of the reactor with the lower portion of the post-reactor and entering said lower portion, preferably near the bottom tangent line, of the post-reactor by means of an internal hydraulic seal (G);
- said hydraulic seal (G) is positioned inside the post-reactor so as to reach the upper portion of the post-reactor and including a spillway point (PS) positioned at a height that corresponds to the midpoint between the position of the outlet nozzle of the liquid phase from the reactor and the position of the inlet nozzle of the liquid phase into the post-reactor plus or minus 20%, preferably plus or minus 10% of said height;
- said post-reactor is positioned in said section below the reactor, said position being such that the position of the spillway point of the hydraulic seal inside the post-reactor is positioned at a height which corresponds to the midpoint between the position of the outlet nozzle of the liquid phase from the reactor and the position of the inlet nozzle of the liquid phase into the post-reactor plus or minus 20%, preferably plus or minus 10% of said height.

The present invention also relates to a balancing and compensation process of the pressure differences (P1, P2) that are generated between a reactor and a post-reactor of a reaction section of an apparatus for the production of melamine as the one of according to the present invention, when the two gas phases leaving the reactor and post-reactor are treated separately, in particular in the case of scrubbing with urea, said process comprising the following steps:

a) urea is reacted in the reactor to give a liquid phase comprising molten melamine, $CO_2$ and $NH_3$, and a gaseous phase comprising $CO_2$, $NH_3$ and melamine in vapour phase;
b) the liquid phase is fed from the reactor to the post-reactor by means of a connection means (M), in which the liquid phase coming from the reactor separates into a liquid phase in the lower portion and a mixed phase (liquid/gas) in the upper portion of said connection means (M);
c) the liquid phase in the lower portion of the connection means (M) between the reactor and the post-reactor is fed to the post-reactor by means of a hydraulic seal inside the post-reactor itself in which the liquid phase rises upwards to the spillway point i.e. to the outlet nozzle of the hydraulic seal;
d) said liquid phase leaving the spillway is caused to enter the post-reactor;
e) the gas phase of the reactor is sent to the scrubbing phase with molten urea or with aqueous solution, recovering the melamine contained therein and sending the scrubbed gases, called off-gases, to external plants;

f) the gas phase of the post-reactor is sent to the sections downstream of the plant for its absorption and recovery within the melamine plant;

said balancing and compensation of the pressure differences P1 and P2 inside the reactor (P1) and the post-reactor (P2) being achieved by moving and positioning the interface between the liquid phase and the mixed phase that moves in the connection means (M), creating a variable liquid seal suitable for compensating the pressure differences between P1 and P2.

The apparatus according to the present invention can comprise from one to three shut-off valves (A, B, C) on the means (T1, T2) when communicating, respectively suitable for transferring the gas phase from the reactor to the scrubbing section and the gas phase from the post-reactor to sections downstream, it preferably comprises three shut-off valves wherein the first shut-off valve (A) is positioned downstream of the intersection point between said means (T1), suitable for transferring the gas phase from the reactor to the scrubbing section, and the outlet duct (U1) of the gas phase from the reactor, the second shut-off valve (B) is positioned between the intersection point between said means (T1) and the outlet duct (U1) of the gas phase from the reactor and the intersection point between the means (T2), suitable for transferring the gas phase from the post-reactor to sections downstream, and the outlet duct (U2) of the gas phase from the post-reactor and the third shut-off valve (C) is positioned downstream of the intersection point between said means (T2), suitable for transferring the gas phase from the post-reactor to sections downstream, and the outlet duct (U2) of the gas phase from the post-reactor.

Said means (T1, T2) are preferably a pipe.

In an apparatus with three shut-off valves (A, B, C), said separate pressure-control systems (PC1/CV1, PC2/CV2) are positioned on the means (T1, T2), the first controller PC1/CV1 downstream of the first shut-off valve (A) and the second controller PC2/CV2 downstream of the third shut-off valve (C), respectively.

In an apparatus with a single shut-off valve (B) positioned between the intersection point between said means (T1) and the outlet duct (U1) of the gas phase from the reactor and the intersection point between said means (T2) and the outlet pipe (U2) of the gas phase from the post-reactor, said separate pressure-control systems (PC1/CV1, PC2/CV2) are respectively positioned, the first controller PC1/CV1 downstream of the intersection point between said means (T1) and the outlet duct (U1) of the gas phase from the reactor and the second controller PC2/CV2 downstream of the intersection point between said means (T2), suitable for transferring the gas phase from the post-reactor to sections downstream, and the outlet pipe (U2) of the gas phase from the post-reactor.

The connection means (M) between reactor and post-reactor is preferably a pipe suitable for connecting the outlet nozzle of the liquid phase from the reactor to the inlet nozzle of said liquid phase into the post-reactor. This pipe can have any configuration depending on the layout of the system, preferably vertical.

The hydraulic seal can be a bulkhead or a pipe.

In the present description, for the illustration of the figures, identical reference numbers or letters are used for indicating construction elements with the same function. Furthermore, for clarity of illustration, some references may not have been repeated in all of the figures.

Indications such as "vertical" and "horizontal", "upper" and "lower" (in the absence of other indications) should be read with reference to the assembly (or operating) conditions and referring to the normal terminology used in current language, wherein "vertical" indicates a direction substantially parallel to that of the force of gravity vector "g" and a horizontal direction perpendicular to it, coinciding with the " horizon direction".

In the present description, the term "upper", "lower" always refers to a higher or lower position with respect to the vertical direction.

In the present description, the term "upper portion" of the reactor refers to the area defined between the end of the tube bundle and the upper flange of the reactor.

In the present description, the term "upper portion" of the post-reactor refers to the portion of the post-reactor situated above 2/3 of the total height of the post-reactor itself.

In the present description, the term "upper portion" of the connection pipe (M) refers to the portion of the pipe between the reactor outlet and the interface between the liquid phase and the mixed phase.

In the present description, the term "lower portion" of the connection pipe (M) means the portion of the pipe between the interface between the liquid phase and the mixed phase and the inlet nozzle of the post-reactor.

In the present description, the term "lower portion" of the post-reactor refers to the portion of the post-reactor situated below 1/3 of the total height of the post-reactor.

In the present description, the term "descending portion of the connection means (M)" refers to the portion of the pipe between the outlet nozzle of the reactor and the inlet nozzle of the post-reactor.

In the present description, the term "tangent-line of the post-reactor bottom" refers to the point where the cylindrical part of the post-reactor structure ends, to which the bottom of the post-reactor itself is welded.

In the present description, the term "downstream" refers to a position in a means or pipe that follows with respect to the normal movement direction of the flow of the stream passing through a given means or pipe.

In the present description, the term "upstream" refers a position in a means or pipe that precedes with respect to the normal movement direction of the flow of the stream passing through a given means or pipe.

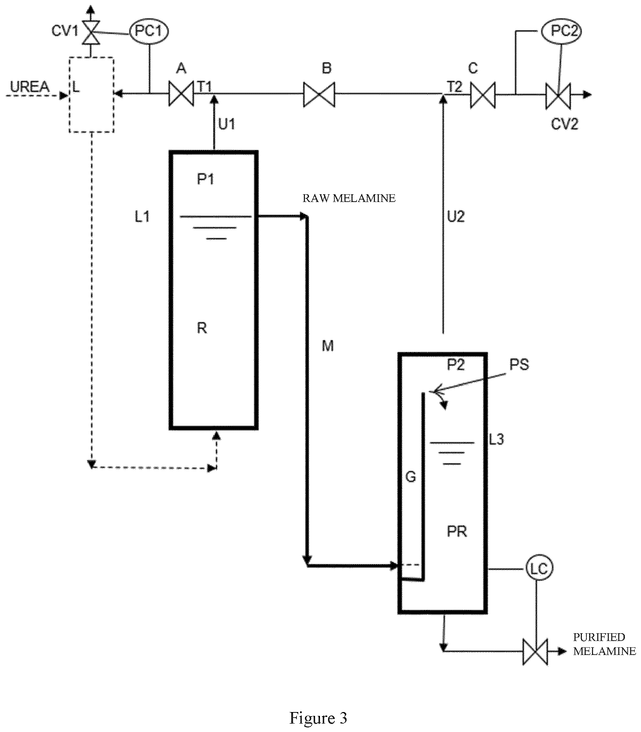

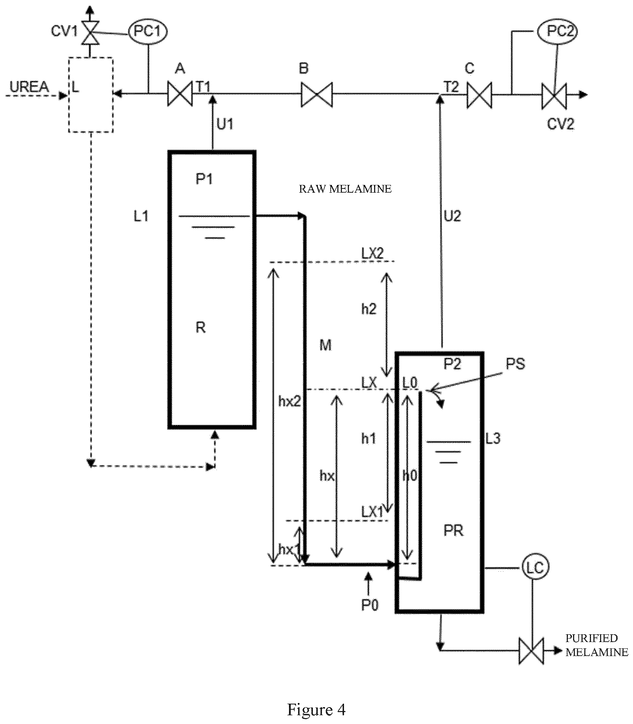

Figures 3 and 4 are representative of the solution according to the present invention, figure 3 schematically and figure 4 in greater detail.

The legend of figure 4 is provided hereunder:

P0 = equilibrium point between gas P + hydraulic P of reactor and post-reactor ($kg/cm^2$)

P1 = reactor gas phase pressure ($kg/cm^2$)

P2 = post-reactor gas phase pressure ($kg/cm^2$)

d = liquid phase density ($kg/cm^3$)

L0 = L2 = spillway level

L1 = liquid level in the reactor

L3 = liquid level in the post-reactor

LX = liquid level in the feed pipe. When P1 = P2 LX = L0

LX1 = liquid level in the feed pipe. When P1>P2 LX1<L0

LX2 = liquid level in the feed pipe. When P1<P2 LX2>L0

hx = height of liquid column LX level (cm)

h0 = height of liquid column from hydraulic seal inlet to spillway (cm)

h1 = level difference between L0 and LX1 (cm)

h2 = level difference between L0 and LX2 (cm)

hx1 = level height LX1 (cm)

hx2 = level height LX2 (cm)

The connection means or pipe (M) has a mixed liquid/gas phase (biphasic) in the upper portion and only a liquid phase (monophasic) in the lower portion. In particular, as can be seen from figure 4, when P1 = P2 the separation line between the mixed liquid/gas phase (biphasic) and the liquid phase (monophasic) is situated at a height (hx) of the connection pipe ( M) equal to the height (h0) of the head or spillway point of the hydraulic seal measured with respect to the point P0 of the pipe (M).

In figure 4, it is evident from a comparison with figures 1 and 2 of the state of the art that:

- the level of the liquid phase inside the reactor is positioned at the height of the outlet nozzle of the reactor itself (L1);
- the spillway point of the hydraulic seal is positioned above the level of the liquid phase in the post-reactor (L3);
- three shut-off valves defined with A, B and C have been inserted in the means (T1, T2).

In the embodiment illustrated in figure 4, the apparatus according to the present invention therefore allows the two gas phases coming from the reactor and from the post-reactor to be conveyed separately to two distinct treatments.

This solution is obtained with the following configuration of the interception and pressure-control devices: PC1/CV1 inserted - valve A open - valve B closed - valve C open - PC2/CV2 inserted.

This mode of use of the apparatus according to the present invention allows the two gas phases to be treated separately and, in particular:

- the gas phase leaving the reactor, rich in $CO_2$, is sent for scrubbing which can be carried out with both molten urea and with aqueous solution according to the scrubbing process used in the scrubbing section, recovering the melamine contained therein and then sending the gases, now called off-gases, to the battery limits to be used in external plants (for example urea plants, not shown in the figure);
- the gas phase leaving the post-reactor, with a low $CO_2$ content, is sent to the sections of the plant downstream (not shown in the figure), which can vary according to the different processes, for its absorption and recovery inside the plant for the production of melamine.

In this case, the pressure of the reactor is regulated and controlled by PC1/CV1 whereas the pressure of the post-reactor is regulated and controlled by PC2/CV2; the two pressures may therefore be subject to small differences due to the use of two different control groups and as the gas phases of the reactor and post-reactor are no longer in communication with each other, the only point of connection between reactor and post-reactor consists in the connection pipe (M) where the balancing of the pressures (P1, P2) takes place with difference = 0 at its lowest point (point P0).

Considering the reactor and post-reactor system under stable running, this configuration of the apparatus allows a simple and effective compensation of a possible pressure variation through the automatic variation of the positioning of the interface between the liquid phase and the mixed phase that is moving in the connection means or pipe (M), creating a liquid seal capable of compensating for the pressure differences between P1 and P2, i.e. the positioning of the liquid column in the connection pipe between reactor and post-reactor through which the melamine produced in the reactor flows into the post-reactor as:

- the melamine enters the outlet nozzle of the liquid phase of the reactor, descends along the upper portion of the pipe in which the mixed liquid/gas phase is present, biphasic area, until it reaches the surface of the liquid phase (i.e. the interface), monophasic area, pushing the liquid phase towards the post-reactor into which said liquid phase enters from the inlet nozzle positioned on the bottom of the hydraulic seal, rises inside the same and exits from its head (specifically from the spillway point); in this way, the hydraulic seal of the post-reactor will be filled with liquid up to the spillway point ensuring a constant level inside (L0).

In this mode of use of the apparatus according to the present invention, three different situations can occur:

i) pressure of the reactor (P1) equal to the post-reactor pressure (P2);
ii) pressure of the reactor (P1) higher than post-reactor pressure (P2) and
iii) pressure of the reactor (P1) lower than post-reactor pressure (P2).

i) Pressure of the reactor (P1) equal to the post-reactor pressure (P2).

In this case, as the pressures in gas phase in the reactor and post-reactor are equal (P1 = P2), the two liquid columns that generate a hydraulic pressure at the point P0 will be in equilibrium so that, as they are composed of the same liquid and therefore have the same density, they will necessarily have the same height and therefore LX will be equal to L0; the melamine further produced inside the reactor will flow through the outlet nozzle and descend along the biphasic area of the connection pipe (M), reaching the interface with the liquid phase which forms the internal liquid column of the pipe (M), inducing an increase in level which corresponds to an immediate exiting of an equal quantity of liquid phase from the top of the hydraulic seal, restoring the level parity.
There will therefore be the situation P1 = P2 and hx = h0 and therefore LX = L0.
The concept can be summarized, again with reference to figure 4, as follows:

$$P1 + (hx \bullet d) = P2 + (h0 \bullet d)$$

wherein d is the density of the liquid, with a total pressure equilibrium, gaseous plus hydraulic, between reactor and post-reactor, at point P0.

ii) Pressure of the reactor (P1) higher than post-reactor pressure (P2)

In this case, the liquid column present in the lower portion (monophasic area) of the connection pipe (M) between reactor and post-reactor will lower by a height (h1) such as to decrease its hydraulic pressure at point P0, compensating for the difference pressure between P1 and P2, in order to have an equal total pressure, gaseous plus hydraulic, between reactor and post-reactor at point P0.
The concept can be summarized, again with reference to figure 4, as follows:

$$P1 + (hx1 \bullet d) = P2 + (h0 \bullet d)$$

iii) Pressure of the reactor (P1) lower than post-reactor pressure (P2).

In this case, the liquid column present in the connection pipe (M) between the reactor and the post-reactor, continuously fed by the melamine leaving the reactor, will rise by a height (h2) such as to increase its hydraulic pressure at the point P0, compensating for the pressure difference between P1 and P2, in order to have an equal total pressure, gaseous plus hydraulic, between reactor and post-reactor at point P0.
The concept can be summarized, again with reference to figure 4, as follows:

$$P1 + (hx2 \bullet d) = P2 + (h0 \bullet d\ ).$$

From what is specified above, it is clear that small variations or oscillations in the gaseous pressure between reactor and post-reactor are automatically compensated for by variations in the hydraulic pressure due to the variation in level of the liquid column in the descending portion of the connection pipe (M), in a particularly simple and effective way.

The greater the height of the internal hydraulic seal of the post-reactor, the greater the compensation field will be.

A further advantage of the apparatus and process according to the present invention is their versatility: in addition, in fact, to the above-mentioned possibility of easily and automatically adjusting any pressure differences between reactor and post-reactor in the case of sending the two gas phases for different kinds of treatment, it also allows the two gas phases to be sent jointly for scrubbing with molten urea or scrubbing with aqueous solution, also allowing the two gas phases to be sent jointly no longer to the scrubbing section, but to the sections downstream of the plant; this situation applies in particular in the case of the lack of scrubbing liquid as in the case of a lack of supply of urea to the reactor.

In the case of the joint sending of the two gas phases to scrubbing with molten urea or scrubbing with aqueous solution, the apparatus represented in figure 4 has the following configuration of the interception and pressure-control devices: PC1/CV1 connected - valve A open - valve B open - valve C closed - PC2/CV2 disconnected.

In this mode, the gas phase of the post-reactor, valve C being closed, passes through the open valve B, encounters the gas phase exiting from the reactor and together they pass through the valve A to jointly reach the valve CV1, whose opening is regulated by PC1 which controls the pressure upstream of the valve CV1; in this way, the pressure of the post-reactor and reactor are kept at the same value as the two gas phases are in equilibrium by means of their respective outlet pipes (U1, U2).

As the pressure of the reactor P1 and the pressure of the post-reactor P2 are equal, the height, and therefore the weight, of the liquid column in the connection pipe (M) between the reactor and post-reactor is necessarily equal to the weight, and therefore to the height, of the liquid column inside the hydraulic seal in the post-reactor.

As the height of the liquid column inside the hydraulic seal, under liquid flow conditions, is constant, the only variable is the height of the liquid column inside the reactor/post-reactor connection pipe which will settle at the same height as the spillway point of the hydraulic seal; in this way the equilibrium of equal pressure at point P0 is satisfied.

The two combined gas streams are thus fed to the scrubbing section for the removal of the melamine contained therein and finally sent as off-gas to the battery limits to be used in external plants (for example urea plants, not shown in the figure).

In the case of the joint conveyance of the two gas phases no longer to the gas scrubbing section, but to the sections downstream of the plant, an application that is used in the event of a lack of scrubbing liquid, as in the case of a lack of urea supply to the reactor, the apparatus represents in figure 4 the following configuration of the interception and pressure-control devices:

PC1/CV1 off - valve A closed - valve B open - valve C open - PC2 / CV2 on.

In this mode, the gas phase of the reactor, valve A being closed, passes through the open valve B, encounters the gas phase exiting from the post-reactor and together they pass through the valve C to jointly reach the valve CV2, whose opening is regulated by PC2 which controls the pressure upstream of the valve CV2; in this way, the post-reactor pressure and the reactor pressure are kept at the same value as the two gas phases are in equilibrium through their respective outlet pipes (U1, U2).

As the pressure of the reactor P1 and the pressure of the post-reactor P2 are equal, the height, and therefore the weight, of the liquid column in the connection pipe (M) between the reactor and post-reactor is necessarily equal to the weight, and therefore to the height, of the liquid column inside the hydraulic seal in the post-reactor.

As the height of the liquid column inside the hydraulic seal, under liquid flow conditions, is constant, the only variable is the height of the liquid column inside the reactor/post-reactor connection pipe which will settle at the same height as the spillway point of the hydraulic seal; in this way, the equilibrium of equal pressure at point P0 is satisfied.

The two combined gas streams are thus fed to the sections downstream of the melamine plant (not shown in the figure).

By means of the embodiment of the apparatus according to the present invention shown in figure 4 and object of the present invention, it is therefore possible to operate safely in the three modes described, conveying the gas phase of the reactor and the gas phase of the post-reactor separately, the gas phase of the reactor to scrubbing and the gas phase of the post-reactor to the sections downstream with complete control of pressure variations, and also jointly for scrubbing, or jointly into the sections downstream of the apparatus.

The apparatus and the process according to the present invention are particularly advantageous as they allow pressure differences to be regulated and controlled simply and automatically, thus rebalancing the pressure in the reactor and in the post-reactor so that they are always the same and preventing a small pressure difference between reactor and post-reactor from causing significant problems: when, in fact, the pressure P1 in the reactor is higher than the pressure P2 in the post-reactor (even by a few tenths of a bar), the higher pressure in the reactor pushes the liquid in the reactor downwards until it uncovers the outlet nozzle of the reactor: the post-reactor will then receive a mixed phase of liquid and gas containing higher quantities of $CO_2$ which prevents the correct functioning of the post-reactor.

When the pressure P1 in the reactor is lower than the pressure P2 in the post-reactor (even by a few tenths of a bar), the liquid phase in the hydraulic seal of the post-reactor is pushed downwards by the increased pressure, whereas the level of the liquid phase in the reactor rises, causing the molten melamine to exit together with the gaseous phase, from the head of the reactor.

The apparatus and the process according to the present invention, on the other hand, also allow these small pressure variations (even in the order of tenths of a bar) to be controlled and balanced in a particularly efficient and simple way.

By way of non-limiting example of the present invention, some embodiment examples of the process according to the present invention are provided hereunder.

Example 1

An example of the automatic balancing procedure according to the invention is described in the present example with reference to figure 4, in a system wherein: distance of L1 from the gas outlet nozzle of the reactor (liquid/gas separation area) = 150 cm

length of the vertical section of the pipe M = 1,200 cm
height of hydraulic seal (h0) from P0 to PS (L0) = 600 cm
gas phase pressure of the reactor 80 kg/cm$^2$
gas phase pressure of the post-reactor 80 kg/cm$^2$
liquid density 0.0013 kg/cm$^3$

$$P1 = P2$$

Under this condition of equal pressure between reactor and post-reactor, the two levels L0 and LX were equal, due to the principle of communicating vessels, and therefore a total pressure at point P0, gaseous plus hydraulic, was found to be equal to:

$$P1 + (hx \bullet d) = P2 + (h0 \bullet d)$$

$$80 + (600 \bullet 0.0013) = 80 + (600 \bullet 0.0013)$$

$$80 + 0.78 = 80 + 0.78$$

$$80.78 = 80.78$$

$$P1 > P2$$

A pressure variation between reactor and post-reactor of 0.4 kg/cm$^2$ was therefore introduced wherein the pressure of the reactor (P1) was equal to 80.4 kg/cm$^2$ and the pressure of the post-reactor (P2 ) equal to 80 kg/cm$^2$.

Under these conditions, the greater total pressure on the reactor side which weighs on the point P0, no longer balanced by the total pressure on the post-reactor side, caused a thrust on the surface of the liquid phase inside the pipe M, pushing the liquid column downwards, which was lowered by a height (h1), decreasing the hydraulic pressure at point P0, up to a new height (hx1), thus establishing a new equilibrium level (LX1); in this case the new equilibrium was reached under the following conditions:

$$80.4 + (hx1 \bullet 0.0013) = 80 + (600 \bullet 0.0013)$$

$$80.4 + (hx1 \bullet 0.0013) = 80.78$$

$$hx1 = (80.78 - 80.4) : 0.0013$$

$$hx1 = 292.3076923077$$

$$P1 < P2$$

In a second test, the pressure variation was generated in the opposite direction, causing an initial lowering of the level (L0) of the hydraulic seal which was immediately compensated for and opposed, thus not undergoing variations, by the rise in the level of the liquid phase ( LX) inside the pipe (h2) due to the continuous arrival of the new melamine produced in the reactor up to a new height (hx2) of the level (LX2) thus increasing the hydraulic pressure at point P0 and restoring the balance between the total pressure on the reactor side and the total pressure on the post-reactor side.

In this case, the new equilibrium was reached under the following conditions:

$$80 + ( hx2 \bullet 0.0013) = 80.4 + (600 \bullet 0.0013)$$

$$80 + ( hx2 \bullet 0.0013) = 81.18$$

$$hx2 = (81.18 - 80) : 0.0013$$

$$hx2 = 907.6923077$$

As demonstrated in both cases, the pressure difference of 0.4 $kg/cm^2$ was completely compensated for by the variation in the level of the interface of the two areas (biphasic and monophasic) inside the pipe (M) without this interfering with the liquid-phase levels inside the reactor and post-reactor.

The possibility of compensation is directly proportional to the heights of the hydraulic seal (h0) and of the vertical section of the pipe, or in any case to the difference in height between the point P0 and the spillway point (PS) of the hydraulic seal, in addition to the point P0 and the outlet nozzle of the liquid phase of the reactor.

COMPARATIVE EXAMPLE 2

In the case of a plant according to the state of the art (as shown in figure 1 or figure 2), in the case of a pressure variation equal to that indicated in Example 1, the passage of gas from the reactor to the post-reactor was detected in the case of a higher pressure in the reactor, and, in the opposite case, a rise in the level inside the reactor with the release of melamine together with the gas phase. As has been seen, in order to balance a pressure variation of 0.4 $kg/cm^2$, a variation of about 308 cm of the liquid column is necessary:

$$P1 > P2$$

The level L1, which is in equilibrium with the spillway point of the hydraulic seal (PS), located approximately 60 cm above the inlet nozzle of the hydraulic seal, should have been lowered by 308 cm in order to compensate for the difference in pressure, but after only 60 cm, the outlet nozzle of the molten melamine opened allowing the passage of the gas phase from the reactor to the post-reactor.

$$P1 < P2$$

The level L1, which is about 150 cm from the outlet nozzle of the gas phase, should have been raised by 308 cm, in order to compensate for the pressure difference, but after only 150 cm, the liquid phase reached the outlet nozzle from the gas phase with the release of melamine together with the gases and with the consequent immediate saturation of the scrubbing liquid and clogging of the gas scrubbing section.

In the case of scrubbing with molten urea, as shown in figure 1, this situation was particularly serious and harmful.

**Claims**

1. An apparatus for the production of melamine which comprises a reaction section and a scrubbing section, wherein:

   - said reaction section comprises at least one reactor (R) and at least one post-reactor (PR);
   - said scrubbing section (L) is suitable for scrubbing the gas phase coming from the reactor with an aqueous solution or is suitable for scrubbing the gas phase coming from the reactor with urea;
   - said reactor is connected to the scrubbing section through a means (T1) suitable for transferring the gas phase from the reactor to the scrubbing section;
   - said post-reactor is connected through a means (T2) suitable for transferring the gas phase from the post-reactor to sections downstream;
   - said means T1 and T2 are distinct, or communicating with at least one shut-off valve (B) positioned between the intersection point between said means (T1) and the outlet duct (U1) of the gas phase from the reactor and the intersection point between said means (T2) and the outlet duct (U2) of the gas phase from the post-reactor;
   - said apparatus comprises two separate pressure-controller systems (PC1/CV1, PC2/CV2) suitable for controlling the pressure inside the reactor and inside the post-reactor respectively, each comprising a shut-off element (CV1, CV2 ) and a pressure-control means (PC1, PC2);
   - said reactor and post-reactor are connected through a means (M) suitable for transferring the melamine from said reactor to said post-reactor, said means (M) connecting the upper portion of the reactor with the lower portion of the post-reactor and entering said lower portion, preferably near the bottom tangent line, of the post-reactor by means of an internal hydraulic seal (G);
   - said hydraulic seal (G) is positioned inside the post-reactor so as to reach the upper portion of the post-reactor and including a spillway point (PS) positioned at a height that corresponds to the midpoint between the position of the outlet nozzle of the liquid phase from the reactor and the position of the inlet nozzle of the liquid phase into the post-reactor plus or minus 20%, preferably plus or minus 10% of said height;
   - said post-reactor is positioned in said section below the reactor, said position being such that the position of the spillway point of the hydraulic seal inside the post-reactor is positioned at a height which corresponds to the midpoint between the position of the outlet nozzle of the liquid phase from the reactor and the position of the inlet nozzle of the liquid phase into the post-reactor plus or minus 20%, preferably plus or minus 10% of said height.

2. The apparatus according to claim 1, comprising from one to three shut-off valves (A, B, C) on the means (T1, T2) when communicating, respectively suitable for transferring the gas phase from the reactor to the scrubbing section and the gas phase from the post-reactor to sections downstream.

3. The apparatus according to any of the previous claims, comprising three shut-off valves wherein the first shut-off valve (A) is positioned downstream of the intersection point between said means (T1), suitable for transferring the gas phase from the reactor to the scrubbing section, and the outlet duct (U1) of the gas phase from the reactor, the second shut-off valve (B) is positioned between the intersection point between said means (T1) and the outlet duct (U1) of the gas phase from the reactor and the intersection point between the means (T2), suitable for transferring the gas phase from the post-reactor to sections downstream, and the outlet duct (U2) of the gas phase from the post-reactor and the third shut-off valve (C) is positioned downstream of the intersection point between said means (T2), suitable for transferring the gas phase from the post-reactor to sections downstream, and the outlet duct (U2) of the gas phase from the post-reactor.

4. The apparatus according to any of the previous claims, comprising three shut-off valves (A, B, C), wherein said separate pressure-control systems (PC1/CV1, PC2/CV2) are respectively positioned on the means (T1, T2), the first controller PC1/CV1 downstream of the first shut-off valve (A) and the second controller PC2/CV2 downstream of the third shut-off valve (C).

5. The apparatus according to claim 1, comprising a single shut-off valve (B) positioned between the intersection point between said means (T1) and the outlet duct (U1) of the gas phase from the reactor and the intersection point between said means (T2) and the outlet duct (U2) of the gas phase from the post-reactor, wherein said separate pressure-control systems (PC1/CV1, PC2/CV2) are respectively positioned, the first controller PC1/CV1 downstream of the intersection point between said means (T1) and the outlet duct (U1) of the gas phase from the reactor and the second controller PC2/CV2 downstream of the intersection point between said means (T2), suitable for transferring the gas phase from the post-reactor to sections downstream, and the outlet duct (U2) of the gas phase from the post-reactor.

6. The apparatus according to any of the previous claims, wherein said means (T1, T2) are a pipe.

7. The apparatus according to any of the previous claims, wherein the connection means (M) between reactor and post-reactor is a pipe suitable for connecting the outlet nozzle of the liquid phase from the reactor to the inlet nozzle of said liquid phase in the post-reactor, said pipe having any configuration depending on the layout of the plant, preferably vertical.

8. The apparatus according to any of the previous claims, wherein the hydraulic seal is a bulkhead or a pipe.

9. A balancing and compensation process of the pressure differences (P1, P2) that are generated between a reactor and a post-reactor of a reaction section of an apparatus for the production of melamine, as the one according to claim 1, when the two gas phases leaving the reactor and post-reactor are treated separately, in particular in the case of scrubbing with urea, said process comprising the following steps:

a) urea is reacted in the reactor to give a liquid phase comprising molten melamine, $CO_2$ and $NH_3$, and a gas phase comprising $CO_2$, $NH_3$ and melamine in vapour phase;
b) the liquid phase is fed from the reactor to the post-reactor by means of a connection means (M), in which the liquid phase coming from the reactor separates into a liquid phase in the lower portion and a mixed phase (liquid/gas) in the upper portion of said connection means (M);
c) the liquid phase in the lower portion of the connection means (M) between the reactor and the post-reactor is fed to the post-reactor by means of a hydraulic seal inside the post-reactor itself in which the liquid phase rises upwards to the spillway point i.e. to the outlet nozzle of the hydraulic seal;
d) said liquid phase leaving the spillway is caused to enter the post-reactor;
e) the gas phase of the reactor is sent to the scrubbing phase with molten urea or with aqueous solution, recovering the melamine contained therein and sending the scrubbed gases, called off-gases, to external plants;
f) the gas phase of the post-reactor is sent to the sections downstream of the plant for its absorption and recovery within the melamine plant;

said balancing and compensation of the pressure differences P1 and P2 inside the reactor (P1) and the post-reactor (P2) being achieved by moving and positioning the interface between the liquid phase and the mixed phase that moves in the connection means (M), creating a variable liquid seal suitable for compensating the pressure differences between P1 and P2.

**Patentansprüche**

1. Vorrichtung zur Herstellung von Melamin, die eine Reaktionssektion und eine Waschsektion umfasst, wobei:

- die Reaktionssektion mindestens einen Reaktor (R) und mindestens einen Nachreaktor (PR) umfasst;
- die Waschsektion (L) zum Waschen der aus dem Reaktor kommenden Gasphase mit einer wässrigen Lösung oder zum Waschen der aus dem Reaktor kommenden Gasphase mit Harnstoff geeignet ist;
- der Reaktor ist über ein Mittel (T1), das zum Überführen der Gasphase aus dem Reaktor zur Waschsektion geeignet ist, mit der Waschsektion verbunden;
- der Nachreaktor über ein Mittel (T2), das zum Überführen der Gasphase aus dem Nachreaktor zu stromabwärts gelegenen Sektionen geeignet ist, verbunden ist;
- die Mittel T1 und T2 getrennt sind oder mit mindestens einem Absperrventil (B) kommunizieren, das zwischen dem Schnittpunkt zwischen den Mitteln (T1) und dem Auslasskanal (U1) der Gasphase aus dem Reaktor und dem Schnittpunkt zwischen den Mitteln (T2) und dem Auslasskanal (U2) der Gasphase aus dem Nachreaktor positioniert ist;
- die Vorrichtung zwei separate Drucksteuersysteme (PC1/CV1, PC2/CV2) umfasst, die zur Steuerung des Drucks im Reaktor und im Nachreaktor geeignet sind und jeweils ein Absperrelement (CV1, CV2) und Drucksteuermittel (PC1, PC2) umfassen;
- der Reaktor und der Nachreaktor durch ein Mittel (M) verbunden sind, das geeignet ist, das Melamin aus dem Reaktor zum Nachreaktor zu überführen, wobei das Mittel (M) den oberen Abschnitt des Reaktors mit dem unteren Abschnitt des Nachreaktors verbindet und durch eine interne hydraulische Dichtung (G) in den unteren Abschnitt, vorzugsweise in der Nähe der unteren Tangente, des Nachreaktors eintritt;
- die hydraulische Dichtung (G) innerhalb des Nachreaktors positioniert ist, um den oberen Abschnitt des Nachreaktors zu erreichen, und einen Überlaufpunkt (PS) beinhaltet, der auf einer Höhe positioniert ist, die dem Mittelpunkt zwischen der Position der Auslassdüse der Flüssigphase aus dem Reaktor und der Position der Einlassdüse der Flüssigphase in den Nachreaktor plus oder minus 20 %, vorzugsweise plus oder minus 10 % dieser Höhe entspricht;
- der Nachreaktor in der Sektion unterhalb des Reaktors positioniert ist, wobei die Position so ist, dass die Position des Überlaufpunkts der hydraulischen Dichtung innerhalb des Nachreaktors auf einer Höhe positioniert ist, die dem Mittelpunkt zwischen der Position der Auslassdüse der Flüssigphase aus dem Reaktor und der Position der Einlassdüse der Flüssigphase in den Nachreaktor plus oder minus 20 %, vorzugsweise plus oder minus 10 % dieser Höhe entspricht.

2. Vorrichtung nach Anspruch 1, umfassend ein bis drei Absperrventile (A, B, C) an den Mitteln (T1, T2) beim Kommunizieren, die jeweils geeignet sind, die Gasphase vom Reaktor zur Waschsektion und die Gasphase vom Nachreaktor zu stromabwärts gelegenen Sektionen zu überführen.

3. Vorrichtung nach einem der vorhergehenden Ansprüche, umfassend drei Absperrventile, wobei das erste Absperrventil (A) stromabwärts des Schnittpunkts zwischen dem Mittel (T1), das zum Überführen der Gasphase aus dem Reaktor zur Waschsektion geeignet ist, und dem Auslasskanal (U1) der Gasphase aus dem Reaktor positioniert ist, das zweite Absperrventil (B) zwischen dem Schnittpunkt zwischen dem Mittel (T1) und dem Auslasskanal (U1) der Gasphase aus dem Reaktor und dem Schnittpunkt zwischen dem Mittel (T2), das zum Überführen der Gasphase aus dem Nachreaktor zu stromabwärts gelegenen Sektionen geeignet ist, und dem Auslasskanal (U2) der Gasphase aus dem Nachreaktor positioniert ist, und das dritte Absperrventil (C) stromabwärts des Schnittpunkts zwischen dem Mittel (T2), das zum Überführen der Gasphase aus dem Nachreaktor zu stromabwärts gelegenen Sektionen geeignet ist, und dem Auslasskanal (U2) der Gasphase aus dem Nachreaktor positioniert ist.

4. Vorrichtung nach einem der vorhergehenden Ansprüche, umfassend drei Absperrventile (A, B, C), wobei die separaten Drucksteuersysteme (PC1/CV1, PC2/CV2) jeweils an den Mitteln (T1, T2), der ersten Steuerung PC1/CV1 stromabwärts des ersten Absperrventils (A) und der zweiten Steuerung PC2/CV2 stromabwärts des dritten Absperrventils (C) positioniert sind.

5. Vorrichtung nach Anspruch 1, umfassend ein einzelnes Absperrventil (B), das zwischen dem Schnittpunkt zwischen den Mitteln (T1) und dem Auslasskanal (U1) der Gasphase aus dem Reaktor und dem Schnittpunkt zwischen den Mitteln (T2) und dem Auslasskanal (U2) der Gasphase aus dem Nachreaktor positioniert ist, wobei die separaten Drucksteuersysteme (PC1/CV1, PC2/CV2) jeweils positioniert sind, die erste Steuerung PC1/CV1 stromabwärts des Schnittpunkts zwischen den Mitteln (T1) und dem Auslasskanal (U1) der Gasphase aus dem Reaktor und die zweite Steuerung PC2/CV2 stromabwärts des Schnittpunkts zwischen den Mitteln (T2), die zum Überführen der Gasphase aus dem Nachreaktor zu stromabwärts gelegenen Sektionen geeignet sind, und dem Auslasskanal (U2) der Gasphase aus dem Nachreaktor.

6. Vorrichtung nach einem der vorhergehenden Ansprüche, wobei die Mittel (T1, T2) ein Rohr sind.

7. Vorrichtung nach einem der vorhergehenden Ansprüche, wobei das Verbindungsmittel (M) zwischen Reaktor und Nachreaktor ein Rohr ist, das geeignet ist, die Auslassdüse der Flüssigphase aus dem Reaktor mit der Einlassdüse der Flüssigphase im Nachreaktor zu verbinden, wobei das Rohr je nach Auslegung der Anlage eine beliebige Konfiguration aufweist, vorzugsweise vertikal.

8. Vorrichtung nach einem der vorhergehenden Ansprüche, wobei die hydraulische Dichtung ein Schott oder ein Rohr ist.

9. Ausgleichs- und Kompensationsverfahren der Druckdifferenzen (P1, P2), die zwischen einem Reaktor und einem Nachreaktor einer Reaktionssektion einer Vorrichtung zur Herstellung von Melamin erzeugt werden, wie nach Anspruch 1, wenn die zwei den Reaktor und den Nachreaktor verlassenden Gasphasen getrennt behandelt werden, insbesondere im Falle einer Wäsche mit Harnstoff, wobei das Verfahren die folgenden Schritte umfasst:

a) Harnstoff wird im Reaktor zu einer Flüssigphase aus geschmolzenem Melamin, $CO_2$ und $NH_3$ und einer Gasphase aus $CO_2$, $NH_3$ und Melamin in der Dampfphase umgesetzt;

b) die Flüssigphase aus dem Reaktor mittels eines Verbindungsmittels (M) dem Nachreaktor zugeführt wird, in dem sich die aus dem Reaktor kommende Flüssigphase im unteren Abschnitt in eine Flüssigphase und im oberen Abschnitt dieses Verbindungsmittels (M) in eine Mischphase (Flüssigkeit/Gas) getrennt wird;

c) die Flüssigphase im unteren Abschnitt des Verbindungsmittels (M) zwischen dem Reaktor und dem Nachreaktor dem Nachreaktor mittels einer hydraulischen Dichtung innerhalb des Nachreaktors selbst zugeführt ist, in dem die Flüssigphase nach oben zum Überlaufpunkt, d. h. zur Auslassdüse der hydraulischen Dichtung, aufsteigt;

d) die Flüssigphase, die den Überlauf verlässt, wird veranlasst, in den Nachreaktor einzutreten;

e) die Gasphase des Reaktors mit geschmolzenem Harnstoff oder mit wässriger Lösung in die Waschphase gesendet wird, wobei das darin enthaltene Melamin zurückgewonnen und die gewaschenen Gase, sogenannte Abgase, an externe Anlagen gesendet werden;

f) die Gasphase des Nachreaktors den der Anlage nachgeschalteten Sektionen zu deren Absorption und Rückgewinnung innerhalb der Melaminanlage zugeführt wird;

wobei das Ausgleichen und Kompensieren der Druckdifferenzen P1 und P2 innerhalb des Reaktors (P1) und des Nachreaktors (P2) durch Bewegen und Positionieren der Grenzfläche zwischen der Flüssigphase und der gemischten Phase, die sich in den Verbindungsmitteln (M) bewegt, erreicht wird, wodurch eine variable Flüssigkeitsdichtung geschaffen wird, die zum Kompensieren der Druckdifferenzen zwischen P1 und P2 geeignet ist.

**Revendications**

1. Appareil pour la production de mélamine qui comprend une section de réaction et une section de lavage, dans lequel :

   - ladite section de réaction comprend au moins un réacteur (R) et au moins un post-réacteur (PR) ;
   - ladite section de lavage (L) est adaptée au lavage de la phase gazeuse provenant du réacteur avec une solution aqueuse ou est adaptée au lavage de la phase gazeuse provenant du réacteur avec de l'urée ;
   - ledit réacteur est relié à la section de lavage par un moyen (T1) permettant de transférer la phase gazeuse du réacteur à la section de lavage ;
   - ledit post-réacteur est relié par un moyen (T2) adapté au transfert de la phase gazeuse du post-réacteur vers les sections en aval ;
   - lesdits moyens T1 et T2 sont distincts ou communiquent avec au moins une vanne d'arrêt (B) positionnée entre le point d'intersection entre ledit moyen (T1) et le conduit de sortie (U1) de la phase gazeuse du réacteur et le point d'intersection entre ledit moyen (T2) et le conduit de sortie (U2) de la phase gazeuse du post-réacteur ;
   - ledit appareil comprend deux systèmes distincts de contrôle de la pression (PC1/CV1, PC2/CV2) pouvant contrôler la pression à l'intérieur du réacteur et à l'intérieur du post-réacteur respectivement, chacun comprenant un élément d'arrêt (CV1, CV2) et un moyen de contrôle de la pression (PC1, PC2) ;
   - ledit réacteur et le post-réacteur sont reliés par un moyen (M) pouvant transférer de la mélamine dudit réacteur vers ledit post-réacteur, ledit moyen (M) reliant la partie supérieure du réacteur à la partie inférieure du post-réacteur et pénétrant dans ladite partie inférieure, de préférence près de la ligne tangente inférieure, du post-réacteur au moyen d'un joint hydraulique interne (G) ;
   - ledit joint hydraulique (G) est positionné à l'intérieur du post-réacteur de manière à atteindre la partie supérieure du post-réacteur et comprend un point de déversement (PS) positionné à une hauteur qui correspond au point médian entre la position de la buse de sortie de la phase liquide du réacteur et la position de la buse d'entrée de la phase liquide dans le post-réacteur plus ou moins 20 %, de préférence plus ou moins 10 % de ladite hauteur ;
   - ledit post-réacteur est positionné dans ladite section en dessous du réacteur, ladite position étant telle que la position du point de déversement du joint hydraulique à l'intérieur du post-réacteur est positionnée à une hauteur qui correspond au point médian entre la position de la buse de sortie de la phase liquide du réacteur et la position de la buse d'entrée de la phase liquide dans le post-réacteur plus ou moins 20 %, de préférence plus ou moins 10 % de ladite hauteur.

2. Appareil selon la revendication 1, comprenant de une à trois vannes d'arrêt (A, B, C) sur les moyens (T1, T2) en communication, respectivement pouvant transférer la phase gazeuse du réacteur vers la section de lavage et la phase gazeuse du post-réacteur vers les sections en aval.

3. Appareil selon l'une quelconque des revendications précédentes, comprenant trois vannes d'arrêt dans lesquelles la première vanne d'arrêt (A) est positionnée en aval du point d'intersection entre ledit moyen (T1), pouvant transférer la phase gazeuse du réacteur vers la section de lavage, et le conduit de sortie (U1) de la phase gazeuse du réacteur, la deuxième vanne d'arrêt (B) est positionnée entre le point d'intersection entre ledit moyen (T1) et le conduit de sortie (U1) de la phase gazeuse du réacteur et le point d'intersection entre le moyen (T2), pouvant transférer la phase gazeuse du post-réacteur vers les sections en aval, et le conduit de sortie (U2) de la phase gazeuse du post-réacteur et la troisième vanne d'arrêt (C) est positionnée en aval du point d'intersection entre ledit moyen (T2), pouvant transférer la phase gazeuse du post-réacteur vers les sections en aval, et le conduit de sortie (U2) de la phase gazeuse du post-réacteur.

4. Appareil selon l'une quelconque des revendications précédentes, comprenant trois vannes d'arrêt (A, B, C), dans lequel lesdits systèmes de contrôle de pression séparés (PC1/CV1, PC2/CV2) sont respectivement positionnés sur les moyens (T1, T2), le premier contrôleur PC1/CV1 en aval de la première vanne d'arrêt (A) et le second contrôleur PC2/CV2 en aval de la troisième vanne d'arrêt (C).

5. Appareil selon la revendication 1, comprenant une seule vanne d'arrêt (B) positionnée entre le point d'intersection entre ledit moyen (T1) et le conduit de sortie (U1) de la phase gazeuse du réacteur et le point d'intersection entre ledit moyen (T2) et le conduit de sortie (U2) de la phase gazeuse du post-réacteur, dans lequel lesdits systèmes séparés de contrôle de la pression (PC1/CV1, PC2/CV2) sont respectivement positionnés, le premier contrôleur PC1/CV1 en aval du point d'intersection entre ledit moyen (T1) et le conduit de sortie (U1) de la phase gazeuse du réacteur et le second contrôleur PC2/CV2 en aval du point d'intersection entre lesdits moyens (T2), pouvant transférer la phase gazeuse du post-réacteur vers les sections en aval, et le conduit de sortie (U2) de la phase gazeuse du post-réacteur.

6. Appareil selon l'une quelconque des revendications précédentes, dans lequel lesdits moyens (T1, T2) sont un tuyau.

7. Appareil selon l'une quelconque des revendications précédentes, dans lequel le moyen de connexion (M) entre le réacteur et le post-réacteur est un tuyau permettant de relier la buse de sortie de la phase liquide du réacteur à la buse d'entrée de ladite phase liquide dans le post-réacteur, ledit tuyau ayant une configuration quelconque en fonction de la disposition de l'installation, de préférence verticale.

8. Appareil selon l'une quelconque des revendications précédentes, dans lequel le joint hydraulique est une cloison ou un tuyau.

9. Procédé d'équilibrage et de compensation des différences de pression (P1, P2) qui sont générées entre un réacteur et un post-réacteur d'une section de réaction d'un appareil de production de mélamine, tel que celui selon la revendication 1, lorsque les deux phases gazeuses sortant du réacteur et du post-réacteur sont traitées séparément, en particulier dans le cas d'un lavage à l'urée, ledit procédé comprenant les étapes suivantes :

a) l'urée réagit dans le réacteur pour donner une phase liquide comprenant de la mélamine fondue, du $CO_2$ et du $NH_3$, et une phase gazeuse comprenant du $CO_2$, du $NH_3$ et de la mélamine en phase vapeur ;
b) la phase liquide est acheminée du réacteur au post-réacteur au moyen d'un moyen de connexion (M), dans lequel la phase liquide provenant du réacteur se sépare en une phase liquide dans la partie inférieure et une phase mixte (liquide/gaz) dans la partie supérieure dudit moyen de connexion (M) ;
c) la phase liquide dans la partie inférieure du moyen de connexion (M) entre le réacteur et le post-réacteur est acheminée vers le post-réacteur au moyen d'un joint hydraulique à l'intérieur du post-réacteur lui-même, dans lequel la phase liquide remonte vers le point de déversement, c'est-à-dire vers la buse de sortie du joint hydraulique ;
d) ladite phase liquide quittant le déversoir est amenée à entrer dans le post-réacteur ;
e) la phase gazeuse du réacteur est envoyée en phase de lavage avec de l'urée fondue ou une solution aqueuse, ce qui permet de récupérer la mélamine qu'elle contient et d'envoyer les gaz lavés, appelés effluents gazeux, vers des installations externes ;
f) la phase gazeuse du post-réacteur est envoyée dans les sections en aval de l'installation pour être absorbée et récupérée dans l'installation de mélamine ;

ledit équilibrage et la compensation des différences de pression P1 et P2 à l'intérieur du réacteur (P1) et du post-réacteur (P2) sont obtenus en déplaçant et en positionnant l'interface entre la phase liquide et la phase mélangée qui se déplace dans le moyen de connexion (M), créant un joint liquide variable adapté à la compensation des différences de pression entre P1 et P2.

Figure 1

Figure 2

17

Figure 3

Figure 4

**EP 4 091 707 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3597641 B1
- DE 10319902 A1
- US 2011054169 A1
- CN 109761922 A
- US 6870050 B2